# EUROPÄISCHE PATENTANMELDUNG

(11) **EP 0 940 413 A2**
(43) Veröffentlichungstag der Anmeldung: **08.09.1999**
(21) Anmeldenummer: 99104112.0
(22) Anmeldetag: 02.03.1999
(51) Int. Cl.: C08F 36/04

(54) **Verfahren zur Inhibierung des Popcornpolymerwachstums**

(30) Priorität: 05.03.1998 DE 19809541
(71) Anmelder: BASF AKTIENGESELLSCHAFT, 67056 Ludwigshafen (DE)
(72) Erfinder: Aron, Maik, 67251 Freinsheim (DE); Bachtler, Michael Dr., 67435 Neustadt (DE); Kanand, Jürgen Dr., 67098 Bad Dürkheim (DE)

(57) **Zusammenfassung**

Verfahren zur Inhibierung des Popcornpolymerwachstums in nichtstabilisierten, zur Bildung von Popcornpolymerisaten neigenden, olefinisch ungesättigte organische Verbindungen enthaltenden Materialien, in dem man einem solchen Material eine wirksame Menge eines aliphatischen Alkohols der Formel I

ROH I

zusetzt, in der R für eine geradkettige, verzweigte oder cyclische C₃- bis C₂₀-Alkyl- oder Alkylengruppe steht, wobei die Alkylengruppe eine zweite Hydroxygruppe trägt.

## Beschreibung

Die vorliegende Erfindung betrifft ein Verfahren zur Inhibierung des Popcornpolymerwachstums in nicht-stabilisierten, zur Bildung von Popcornpolymeren neigenden, olefinisch ungesättigte, organische Verbindungen enthaltenden Materialien.

Viele olefinisch ungesättigte organische Monomere, z.B. Styrol und insbesondere Diene mit konjugierten Doppelbindungen, wie 1,3-Butadien oder Isopren, neigen zur spontanen unerwünschten Bildung von wegen ihres Erscheinungsbildes so genannter Popcornpolymere, beispielsweise bei der Lagerung und dem Transport dieser Monomere, deren Gewinnung oder Weiterverarbeitung. Bei diesen Popcornpolymeren handelt es sich meist um stark vernetzte, unlösliche Massen, die sich in Form schaumiger, krustiger Polymerisat-Körner mit Blumenkohl-artiger Struktur an den Wandungen von Tanks, Rohrleitungen, Apparaten und Reaktoren absetzen. Die unerwünschte Popcornpolymerisation kann durch die Einwirkung unterschiedlicher Faktoren auf das betreffende Monomer ausgelöst werden, beispielsweise durch Sauerstoff, Hitze und Rost als auch durch im Monomer bereits enthaltene Popcornpolymerteilchen, die die Popcornpolymerbildung katalysieren.

Besonders kritisch erweist sich die Popcornpolymerbildung bei konjugierten Dien-Monomeren, wie 1,3-Butadien oder Isopren. Hier kann es durch Popcornpolymerisation zum Zusetzen und Verstopfen von Rohrleitungen und Reaktoren, zum Durchpolymerisieren von Tankfüllungen und in der Folge zum Bersten der betreffenden Vorrichtungen kommen.

Zur Hemmung dieser Popcornpolymerisation setzt man den zur Bildung von Popcornpolymerisaten neigenden Monomeren bereits im Zuge von deren Herstellung Stabilisatoren zu, die die Bildung solcher Popcornpolymere bei der Herstellung, der Lagerung, dem Transport und der Weiterverarbeitung dieser Monomere unterdrücken oder zumindest verlangsamen. Die von der Produktionseinrichtung kommenden betreffenden Monomere als auch die im Handel befindlichen Monomere enthalten deshalb, wenn nichts anderes explizit angegeben ist, grundsätzlich solche Stabilisatoren.

Im Falle von 1,3-Butadien werden zur Vermeidung der Popcornpolymerisation üblicherweise Radikalfänger, wie 4-tert.Butylbrenzcatechin (TBC) oder 2,6-Di-tert.butyl-p-kresol, eingesetzt (vgl. Ullmann's Encyclopedia of Industrial Chemistry; 5th Ed., Vol. A4, S. 431-446, VCH-Verlagsgesellschaft, Weinheim 1985). Außer diesen und ähnlichen als Radikalfänger bekannten Phenolen werden im Stand der Technik auch Natriumnitrit (China Synthetic Rubber Industry 11, 357 (1988), Schwefelverbindungen, wie Schwefelkohlenstoff, Schwefelwasserstoff, Alkanthiole oder organische Disulfide, aber auch elementarer Phosphor (US-A 4 404 413) genannt. Ebenso werden im Stand der Technik außer Natriumnitrit noch eine Reihe anderer stickstoffhaltiger Popcornpolymerisations-Inhibitoren genannt, z.B. N,N-Dialkylhydroxylamine, Trialkylaminoxide (JP-A 223 003 (1988)), Nitrosoverbindungen, NO₂, N₂O₃, aromatische Amine, Hydroxylamine (USA-A 3 148 225), N-Hydroxymorpholin (USA-A 3 265 752), N-Hydroxypiperidin (US-A 3 265 751), Addukte aus Phenolen und Hydroxylaminen (US-A 3 493 603), die Umsetzungsprodukte aus der Reaktion von salpetriger Säure oder NO₂ mit 1,3-Dichlorbut-2-en oder Diisobutylen (US-A 3 560 577), Butyraldoxim (US-A 3 560 577) oder die Umsetzungsprodukte aus der Reaktion von Distickstofftetroxid mit Diisobutylen (US-A 3 175 012). US-A 3 557 232 nennt Triarylmethylchloride als Inhibitoren und in WO 92/12948 werden Alkylhalogenide als Inhibitoren für die Popcornpolymerbildung eingesetzt.

Obgleich die vorgenannten Inhibitoren des Standes der Technik die Bildung von Popcornpolymerisaten inhibieren, erweisen sie sich als problematisch, wenn die betreffende, stabilisierte olefinische Verbindung mit Hilfe metallorganischer Homogenkatalysatoren weiterverarbeitet werden soll, da sich diese Inhibitoren an die Homogenkatalysatoren binden können und auf diese Weise deren Reaktivität und Selektivität bei der zu katalysierenden Umsetzung nachteilig beeinflussen oder gar diese Homogenkatalysatoren inaktivieren können. Der Großteil der Inhibitoren des Standes der Technik vermag zwar die Neubildung von Popcornpolymerisat-Keimen zu unterdrücken, ist aber nicht imstande, die Popcornpolymerisation dann zu unterbinden, wenn sich im betreffenden Olefin bereits Popcornpolymerisate gebildet haben, die sehr wirksam die Popcornbildung katalysieren.

JP-A 222 037 (1983) betrifft ein Verfahren zur Absorption von 1,3-Butadien aus gasförmigen Gemischen, bei dem als geeignetes Absorptionsmittel u.a. Butanol genannt wird. Es wird in dieser Schrift ausdrücklich empfohlen, das Butadien durch Zusatz von TBC zum Absorptionsmittel gegen Popcornpolymerisation zu stabilisieren.

Im Verfahren gemäß WO 95/19334 wird handelsübliches, mit TBC-stabilisiertes 1,3-Butadien u.a. mit Alkoholen zu den betreffenden Allylethern umgesetzt. Bei der Homogen- bzw. Heterogenkatalyse dieser Umsetzung werden die anzuwendenden Homogen- bzw. Heterogenkatalysatoren durch Reaktion mit dem starken Komplexbildner TBC im Laufe der Zeit in ihrer Reaktivität und Selektivität nachteilig beeinflußt oder gar inaktiviert. Das schwerflüchtige, im flüssigen Reaktionsmedium dieses Verfahrens gut lösliche TBC wird außerdem durch das Reaktionsmedium in die weiteren Anlagenteile verschleppt, wo es auf andere verfahrensgemäß durchzuführende, homogen- bzw. heterogenkatalytische Umsetzungen entsprechend nachteilige Auswirkungen hat. Bei der destillativen Abtrennung des nicht-umgesetzten Butadiens vom daraus erzeugten Allylether verbleibt das schwerflüchtige TBC in der flüssigen Phase und es kommt zur Abscheidung von Popcornpolymerisaten aus der Gasphase, die zur Abschaltung und Reinigung der Anlage zwingen. Wird versucht die Popcornpolymerbildung im Gasraum der Destillationsvorrichtung durch kontinuierliches Versprühen von TBC in den Gasraum zu verhindern, wird zwar die Popcornpolymerbildung unterbunden, dies wird jedoch mit dem Nachteil erkauft, daß in den weiteren Verfahrensschritten infolge der erhöhten TBC-Konzentration die Homogen- bzw. Heterogenkatalysatoren noch schneller geschädigt und ihre Standzeit verkürzt wird.

Der vorliegenden Erfindung lag deshalb die Aufgabe zugrunde, ein Verfahren zur Hemmung des Popcornpolymerwachstums, worin die Neubildung von Popcornpolymeren mit eingeschlossen ist, zu finden, das nicht mit den Nachteilen des Standes der Technik behaftet ist. Insbesondere sollten die hierfür eingesetzten Inhibitoren auch in der Lage sein, die durch Popcornpolymerisate katalysierte Popcornbildung zu hemmen und sollten mit einer Vielzahl metallorganischer Homogenkatalysatoren in dem Sinne verträglich sein, daß sie deren katalytisches Verhalten nicht nachteilig beeinflussen.

Dementsprechend wurde ein Verfahren zur Inhibierung des Popcornpolymerwachstums in nicht-stabilisierten, zur Bildung von Popcornpolymerisaten neigenden, olefinisch ungesättigte organische Verbindungen enthaltenden Materialien gefunden, das dadurch gekennzeichnet ist, daß man einem solchen Material eine wirksame Menge eines aliphatischen Alkohols der Formel I

ROH I

zusetzt, in der R für eine geradkettige, verzweigte oder cyclische C₃- bis C₂₀-Alkyl- oder Alkylengruppe steht, wobei die Alkylengruppe eine zweite Hydroxygruppe trägt.

Des Weiteren betrifft die vorliegende Erfindung die Verwendung von aliphatischen Alkoholen der Formel I

ROH I

, in der R eine geradkettige, verzweigte oder cyclische C₃- bis C₂₀-Alkyl- oder Alkylengruppe ist und die Alkylengruppe eine zweite Hydroxygruppe trägt, zur Inhibierung des Popcornpolymerwachstums in nicht-stabilisierten, zur Bildung von Popcornpolymeren neigenden, olefinisch ungesättigte organische Verbindungen enthaltenden Materialien.

Ferner wurden Zusammensetzungen aus nicht-stabilisiertem Butadien und/oder Isopren enthaltendem Material und 0,1 bis 90 Gew.-% eines Alkohols ROH I gefunden.

Für die Durchführung der erfindungsgemäßen Verfahren sind praktisch alle Alkohole ROH I geeignet, in denen R für eine geradkettige, verzweigte oder cyclische Alkylgruppe steht. Dementsprechend können die Alkohole ROH I primäre, sekundäre oder auch tertiäre Alkohole sein. Im allgemeinen werden im erfindungsgemäßen Verfahren C₃- bis C₂₀-Alkohole, vorzugsweise C₄- bis C₁₂-Alkohole und besonders bevorzugt C₄- bis C₆-Alkohole verwendet. Die Verwendung von relativ leichtflüchtigen C₄- bis C₆-Alkoholen kann insbesondere dann von Vorteil sein, wenn die damit versetzte olefinische Verbindung in einer Gasphasenumsetzung weiterverarbeitet wird und hierbei eine Inhibierung der Popcornpolymerbildung erforderlich ist.

Im folgenden wird eine lediglich beispielhafte Aufzählung geeigneter Alkohole ROH I gegeben: Propanol, Isopropanol, n-Butanol, 2-Butanol, Isobutanol, tert.Butanol, n-Pentanol, Amylalkohol, Neopentylalkohol, Cyclopentylalkohol, die Hexanole, wie n-Hexanol oder Cyclohexanol, die Heptanole, Octanole, wie n-Octanol oder 2-Ethylhexanol, die Nonanole, die Decanole, wie n-Decanol oder 2-Propylheptanol, die Eicosanole, Dodecanole, Pentadecanole oder Octadecanole. Anstelle dieser Monoalkohole können auch die entsprechenden Diole, wie 1,2-Propylenglykol, 1,3-Propylenglykol, 1,4-Butandiol, 1,2-Butylenglykol, Neopentylglykol usw. als Alkohole ROH I im erfindungsgemäßen Verfahren eingesetzt werden. Bevorzugt werden im allgemeinen C₄- bis C₁₂-Alkohole im erfindungsgemäßen Verfahren verwendet, besonders bevorzugt die Butanole, insbesondere n-Butanol.

Die im erfindungsgemäßen Verfahren anwendbaren Alkohole bewirken die Inhibierung des Popcornpolymerwachstums in den zur Bildung von Popcornpolymeren neigenden, olefinisch ungesättigte organische Verbindungen enthaltenden Materialien sowohl in der flüssigen Phase als auch, selbstverständlich abhängig von der Flüchtigkeit des betreffenden Alkohols und der angewandten Temperatur, in der Gasphase.

Das erfindungsgemäße Verfahren eignet sich zur Inhibierung des Popcornpolymerwachstums in allen zur Bildung von Popcornpolymerisaten neigenden, olefinisch ungesättigten organischen Verbindungen. Solche olefinisch ungesättigten Verbindungen sind z.B. Styrol sowie die besonders stark zur Bildung von Popcornpolymerisaten neigenden Diene mit konjugierten Doppelbindungen, wie 1,3-Butadien, Isopren oder 2,3-Dimethyl-buta-1,3-dien. Besonders bevorzugt wird das erfindungsgemäße Verfahren zur Inhibierung des Popcornpolymerwachstums in 1,3-Butadien oder Isopren angewandt.

Als nicht-stabilisierte, zur Bildung von Popcornpolymerisaten neigende, olefinisch ungesättigte organische Verbindungen enthaltende Materialien im Sinne dieser Anmeldung werden sowohl die betreffenden Olefine verstanden, die keinen Stabilisator zur Inhibierung des Popcornpolymerwachstums enthalten, als auch Mischungen dieser Stabilisator-freien Olefine mit anderen chemischen Verbindungen, z.B. Reaktionspartnern, Umsetzungsprodukten, Verunreinigungen, Lösungsmitteln und/oder Katalysatoren, die keine Wirkung als Stabilisator zur Hemmung des Popcornpolymerwachstums entfalten. Da es die Aufgabe der vorliegenden Erfindung war, die mit der Verwendung von aus dem Stand der Technik bekannten Inhibitoren der Popcornpolymerisation verbundenen Nachteile zu vermeiden, ist die Verwendung von derart gegen Popcornpolymerisation stabilisierten Materialien nicht Gegenstand der vorliegenden Erfindung.

Für die Zwecke des erfindungsgemäßen Verfahrens kann der Alkohol ROH I dem nicht-stabilisierten zur Popcornpolymerisation neigen-den Olefin-haltigen Materials bereits bei der Isolierung der betreffenden Olefine zugesetzt werden oder aber auch erst beim Transport dieser Olefin-haltigen Materialien einschließlich des Transports zu Lagereinrichtungen oder auch erst bei deren Lagerung. Für den Fall, daß nur ein mit einem Stabilisator zur Inhibierung der Popcornpolymerisation stabilisiertes Olefin-haltiges Material zur Verfügung steht, kann das nicht-stabilisierte, erfindungsgemäß zu stabilisierende Olefin-haltige Material durch destillative, extraktive, adsorptive oder anderweitige Abtrennung des zugesetzten Stabilisators des Standes der Technik erhalten werden und diesem zur Hemmung des Popcornpolymerwachstums der erfindungsgemäß einzusetzende Alkohol ROH I zugesetzt werden. Es versteht sich dabei von selbst, daß anstelle eines einzelnen Alkohols ROH I auch Gemische aus zwei oder mehreren der Alkohole ROH I im erfindungsgemäßen Verfahren eingesetzt werden können.

Der Zusatz des Alkohols ROH I zum nicht-stabilisierten, zur Bildung von Popcornpolymerisaten neigenden, olefinisch ungesättigte organische Verbindungen enthaltenden Material kann diskontinuierlich oder kontinuierlich erfolgen, wobei die Zugabe des Alkohols ROH I vorteilhaft so gesteuert wird, daß die für eine Inhibierung des Popcornpolymerwachstums benötigte wirksame Menge des Alkohols ROH I in diesem Material nicht unterschritten wird. Kommt es im Falle einer länger anhaltenden Unterschreitung dieser wirksamen Menge zur Popcornpolymerbildung, kann das autokatalytische Wachstum dieser Popcornpolymere durch Nachdosieren des Alkohols ROH I inhibiert werden. Eine diskontinuierliche Zugabe des Alkohols ROH I zum nicht-stabilisierten, Olefin-haltigen Material wird in der Regel bei der Tanklagerung oder dem Transport in geschlossenen Behältern bevorzugt, wohingegen zur Inhibierung des Popcornpolymerwachstums im strömenden, nicht-stabilisierten Olefin-haltigen Material, z.B. in Rohrleitungen oder Reaktoren, der Alkohol ROH I bevorzugt kontinuierlich zudosiert wird.

Wenn das zur Bildung von Popcornpolymerisaten neigende, olefinisch ungesättigte organische Verbindungen enthaltende Material in der Gasphase umgesetzt oder destilliert werden soll, kann in der Regel vorteilhaft ein Alkohol ROH I zur Inhibierung des Popcornpolymerwachstums eingesetzt werden, der unter den angewandten Bedingungen eine ausreichende Flüchtigkeit hat, um das Popcornpolymerwachstum aus der Gasphase zu hemmen. Ist die Anwendung eines solchermaßen flüchtigen Alkohols ROH I z.B. aus Gründen der Reaktionsführung oder aufgrund von Trennproblemen unerwünscht, kann das Popcornpolymerwachstum aus der Gasphase in einem solchen Gasphasenreaktor oder Destillationsvorrichtung vorteilhaft dadurch verhindert werden, daß man einen schwererflüchtigen Alkohol ROH I in den Kopf oder oberen Teil der betreffenden Gasphasenapparatur einleitet und z.B. durch Versprühen, im Gasraum dieser Vorrichtung verteilt.

Der Alkohol ROH I wird im allgemeinen dem nicht-stabilisierten, zur Bildung von Popcornpolymerisaten neigenden, olefinisch ungesättigte organische Verbindungen enthaltenden Material in einer Menge von 0,1 Gew.-% bis 90 Gew.-% zugegeben.

Bereits bei Zusatz der geringen Menge von 100 Gew.-ppm Alkohol ROH I wird eine deutliche Verlangsamung des Popcornpolymerwachstums verglichen mit einer nicht-stabilisierten Probe beobachtet, die bei einer voraussehbaren, baldigen Weiterverarbeitung des betreffenden Materials für eine wirksame Inhibierung des Popcornpolymerwachstums ausreichend sein kann. Im Falle einer längeren Lagerung oder einer Lagerung des betreffenden Materials unter nicht-optimalen Bedingungen, z.B. in verschmutzten, rostigen Tanks, kann es zweckmäßig sein, die Konzentration des Alkohols ROH I im betreffenden Material zu erhöhen, desgleichen, wenn in einer Charge des betreffenden Materials bereits Popcornpolymerisation eingesetzt hat und das durch die bereits gebildeten Popcornpolymerisate katalysierte Popcornpolymerwachstum zu inhibieren ist. Für die Zwecke der Lagerung und des Transports werden zur Inhibierung des Popcornpolymerwachstums dem nicht-stabilisierten, zur Bildung von Popcornpolymerisaten neigenden, olefinisch ungesättigte organische Verbindungen enthaltenden Material im allgemeinen 0,1 Gew.-% bis 90 Gew.-%, vorzugsweise 0,5 Gew.-% bis 25 Gew.-% und besonders bevorzugt 1 bis 15 Gew.-% des Alkohols ROH I zugesetzt. Selbstverständlich ist es möglich, größere Mengen an Alkohol ROH I als die zuvor angegebenen dem betreffenden Material zuzusetzen.

Soll das nicht-stabilisierte, zur Bildung von Popcornpolymerisaten neigende, olefinisch ungesättigte organische Verbindungen enthaltende Material bei der Weiterverarbeitung mit einem Alkohol ROH I umgesetzt werden, so bewirkt der als Reaktionspartner zugesetzte Alkohol ROH I gleichzeitig die Inhibierung des Popcornpolymerwachstums des eingesetzten Olefin-haltigen Materials im Reaktor. In einem solchen Fall kann die Menge des zugesetzten, sowohl als Reaktionspartner dienenden als auch als Inhibitor des Popcornpolymerwachstums wirkenden Alkohols ROH I, entsprechend den gewählten Reaktionsbedingungen gleich der Menge des Olefinhaltigen Materials sein oder diese sogar übersteigen, z.B. beim Arbeiten mit einem stöchiometrischen Überschuß des Alkohols ROH I. Beim Einsatz eines mit einem Inhibitor der Popcornpolymerisation ausreichend stabilisierten Olefins wird durch den Zusatz eines Alkohols ROH I im allgemeinen keine zusätzliche Stabilisierung des Olefins hinsichtlich der Popcornpolymerisation beobachtet. Da durch die Verwendung eines Alkohols ROH I zur Inhibierung des Popcornpolymerwachstums die Nachteile der bekannten Inhibitoren der Popcornpolymerbildung erfindungsgemäß vermieden werden sollen, ist die Verwendung eines Alkohols ROH I im Zusammenwirken mit einem Inhibitor des Standes der Technik zur Stabilisierung von Olefin-haltigen Materialien zur Vermeidung der Popcornpolymerisatbildung nicht Gegenstand der vorliegenden Erfindung.

Wie bereits erwähnt, kann das erfindungsgemäße Verfahren besonders vorteilhaft zur Inhibierung des Popcornpolymerwachstums in nicht-stabilisierten, zur Bildung von Popcornpolymerisaten neigenden, olefinisch ungesättigte organische Verbindungen enthaltenden Materialien bei deren Lagerung und Transport eingesetzt werden, wobei das erfindungsgemäße Verfahren bevorzugt zur Inhibierung des Popcornpolymerwachstums in konjugierte Diene, wie 1,3-Butadien oder Isopren oder Gemischen dieser Diene, insbesondere 1,3-Butadien, enthaltenden Materialien angewandt wird. Geeignete erfindungsgemäß zur Inhibierung des Popcornpolymerwachstums bei Lagerung und Transport mit Alkoholen ROH I stabilisierte Zusammensetzungen bestehen in der Regel aus nicht-stabilisiertem 1,3-Butadien und/oder Isopren, einschließlich üblicher herstellungsbedingter Verunreinigungen und 0,1 Gew.-% bis 90 Gew.-%, vorzugsweise 0,5 Gew.-% bis 25 Gew.-% und besonders bevorzugt 1 Gew.-% bis 15 Gew.-% eines oder mehrerer Alkohole ROH I, vorzugsweise n-Butanol.

Besonders vorteilhaft erweist sich das erfindungsgemäße Verfahren zur Inhibierung des Popcornpolymerwachstums, beispielsweise im Verfahren zur Herstellung von Butyraldehyd und/oder n-Butanol gemäß WO 95/19334. Wird das im Verfahren von WO 95/19334 eingesetzte handelsübliche, mit TBC-stabilisierte 1,3-Butadien durch erfindungsgemäß mit einem Alkohol ROH I, insbesondere n-Butanol, stabilisiertes 1,3-Butadien ersetzt, so wirkt sich diese Maßnahme sowohl vorteilhaft auf Ausbeute, Selektivität und Katalysatorstandzeit der in diesem Verfahren angewandten homogen- bzw. heterogenkatalysierten Verfahrensschritte aus.

### Beispiele 1 bis 13

Nicht-stabilisiertes 1,3-Butadien wurde in einem Glasautoklaven mit unterschiedlichen Mengen an n-Butanol versetzt und in Gegenwart eines Initiators der Popcornpolymerisation 2 Monate auf 65°C gehalten. Als Initiator der Popcornpolymerisation wurden in den Proben 1 bis 7 rostige Nägel und in den Proben 8 bis 13 Popcornpolymerisatkeime, die bei Lagerung von nicht-stabilisiertem 1,3-Butadien gebildet worden waren, verwendet. Den Proben 1 und 8, nachfolgend als Blindproben bezeichnet, wurde kein n-Butanol zugesetzt.

Die den einzelnen Proben zugesetzte Menge an n-Butanol wird in der folgenden Tabelle angegeben.

| Probe | n-Butanolgehalt | Keim |
|---|---|---|
| 1 | 0 | Nagel |
| 2 | 100 Gew.-ppm | Nagel |
| 3 | 1000 Gew.-ppm | Nagel |
| 4 | 5000 Gew.-ppm | Nagel |
| 5 | 1 Gew.-% | Nagel |
| 6 | 5 Gew.-% | Nagel |
| 7 | 10 Gew.-% | Nagel |
| 8 | 0 | Popcorn |
| 9 | 1000 Gew.-ppm | Popcorn |
| 10 | 1 Gew.-% | Popcorn |
| 11 | 5 Gew.-% | Popcorn |
| 12 | 10 Gew.-% | Popcorn |
| 13 | 15 Gew.-% | Popcorn |

### Ergebnisse:

Beide Blindproben waren bereits nach 4 Wochen mit Popcornpolymerisat zugewachsen. Die Versuche 1 und 8 wurden daher aus Sicherheitsgründen abgebrochen.

In der Probe mit 100 Gew.-ppm n-Butanol konnte nach 2 Monaten beginnendes Popcornpolymerwachstum festgestellt werden. In den Proben mit 1000 und 5000 Gew.-ppm n-Butanol wurde nach 2 Monaten kein Popcornpolymerisat gefunden, allerdings waren die Proben viskos geworden, was auf gebildete Butadienoligomere zurückzuführen ist. Die Proben mit 1 Gew.-% n-Butanol und mehr waren auch nach 2 Monaten noch dünnflüssig, es konnte weder Popcornpolymerisat- noch Oligomerenbildung festgestellt werden.

### Beispiel 14

Nicht stabilisiertes 1,3-Butadien wurde in einem Glasautoklaven entweder mit 5 Gew.-% n-Butanol oder 5 Gew.-% n-Octanol versetzt und in Gegenwart von Popcornpolymerisat als Initiator der Popcornpolymerisation 1 Monat auf einer Temperatur von 65°C gehalten. Der gleiche Versuch wurde mit einer Blindprobe durchgeführt, der kein Alkohol zugesetzt worden war. Nach 1 Monat konnte in den alkoholhaltigen Proben keine Popcornpolymerisatbildung beobachtet werden, wohingegen in der Blindprobe Popcornpolymerisat in erheblichem Umfang gebildet worden war. In den Proben mit Alkoholzusatz wurde in der Flüssigphase die Bildung geringer Mengen eines von gummiartigem Popcornpolymerisat verschiedenen, wasserklaren, gelförmigen Polymers beobachtet, das nicht die negativen Eigenschaften von Popcornpolymerisat hat.

## Patentansprüche

1. Verfahren zur Inhibierung des Popcornpolymerwachstums in nicht-stabilisierten, zur Bildung von Popcornpolymerisaten neigenden, olefinisch ungesättigte organische Verbindungen enthaltenden Materialien, dadurch gekennzeichnet, daß man einem solchen Material eine wirksame Menge eines aliphatischen Alkohols der Formel I
ROH I
zusetzt, in der R für eine geradkettige, verzweigte oder cyclische C₃- bis C₂₀-Alkyl- oder Alkylengruppe steht, wobei die Alkylengruppe eine zweite Hydroxygruppe trägt.

2. Verfahren nach Anspruch 1, dadurch gekennzeichnet, daß die zur Bildung von Popcornpolymerisaten neigende, olefinisch ungesättigte organische Verbindung eine konjugierte Diengruppierung enthält.

3. Verfahren nach den Ansprüchen 1 und 2, dadurch gekennzeichnet, daß die zur Bildung von Popcornpolymerisaten neigende, olefinisch ungesättigte organische Verbindung 1,3-Butadien ist.

4. Verfahren nach den Ansprüchen 1 und 2, dadurch gekennzeichnet, daß die zur Bildung von Popcornpolymerisaten neigende, olefinisch ungesättigte Verbindung Isopren ist.

5. Verfahren nach den Ansprüchen 1 bis 4, dadurch gekennzeichnet, daß man als aliphatischen Alkohol ROH I n-Butanol verwendet.

6. Verfahren nach den Ansprüchen 1 bis 5, dadurch gekennzeichnet, daß man den Alkohol ROH I dem organischen Material in einer Menge von 0,1 Gew.-% bis 90 Gew.-% zusetzt.

7. Verwendung von aliphatischen Alkoholen der Formel I
ROH I
in der R eine geradkettige, verzweigte oder cyclische C₃- bis C₂₀-Alkyl- oder Alkylengruppe ist und die Alkylengruppe eine zweite Hydroxygruppe trägt, zur Inhibierung des Popcornpolymerwachstums in nicht-stabilisierten, zur Bildung von Popcornpolymerisaten neigenden, olefinisch ungesättigte, organische Verbindungen enthaltenden Materialien.

8. Verwendung von Alkoholen der Formel ROH I gemäß Anspruch 7, dadurch gekennzeichnet, daß man n-Butanol zur Inhibierung des Popcornpolymerwachstums in 1,3-Butadien oder Isopren enthaltenden Materialien verwendet.

9. Zusammensetzungen aus nicht-stabilisiertem Butadien und/oder Isopren enthaltendem Material und 0,1 bis 90 Gew.-% eines Alkohols ROH I gemäß Anspruch 1.
